(19) Europäisches Patentamt
European Patent Office
Office européen des brevets

(11) Veröffentlichungsnummer : **0 166 293**
**B1**

(12) **EUROPÄISCHE PATENTSCHRIFT**

(45) Veröffentlichungstag der Patentschrift : **30.08.89**

(51) Int. Cl.[4] : **C 07 C 51/60**, C 07 C 53/50

(21) Anmeldenummer : **85107160.5**

(22) Anmeldetag : **11.06.85**

# (54) Verfahren zur Herstellung halogenierter aliphatischer Carbonsäurefluoride.

(30) Priorität : **18.06.84 DE 3422576**

(43) Veröffentlichungstag der Anmeldung : **02.01.86 Patentblatt 86/01**

(45) Bekanntmachung des Hinweises auf die Patenterteilung : **30.08.89 Patentblatt 89/35**

(84) Benannte Vertragsstaaten : **BE CH DE FR GB IT LI NL**

(56) Entgegenhaltungen :
**US-A- 1 921 767**
**US-A- 1 965 556**

(73) Patentinhaber : **HOECHST AKTIENGESELLSCHAFT**
**Postfach 80 03 20**
**D-6230 Frankfurt am Main 80 (DE)**

(72) Erfinder : **Siegemund, Günter, Dr.**
**Frankfurter Strasse 21**
**D-6238 Hofheim am Taunus (DE)**
Erfinder : **Schwertfeger, Werner, Dr.**
**Erlenstrasse 8**
**D-6306 Langgöns (DE)**

**EP 0 166 293 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Halogenierte aliphatische Carbonsäurefluoride sind vielfältig einsetzbare Zwischenprodukte. So kann z. B. ω-H-Perfluorpropionsäurefluorid nach folgendem Reaktionsschema in den ω-H-Perfluorpropylvinylether überführt werden :

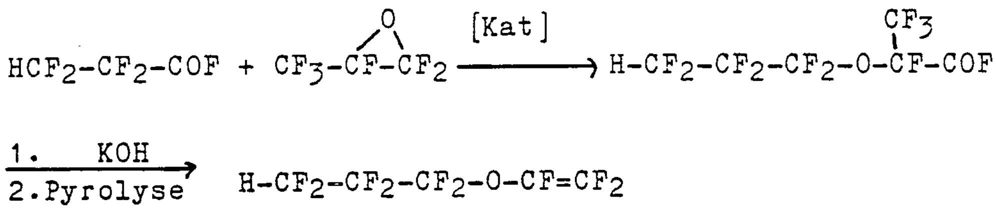


ω-H-Perfluorpropylvinylether ist ein wichtiges Comonomeres zur Herstellung von perfluorierten Ionenaustauscherharzen (vgl. EP-A-0 088 285). Für die Synthese von Carbonsäurefluoriden gibt es verschiedene Möglichkeiten, die meist von den zugrundeliegenden Carbonsäuren ausgehen. Diese Verfahren können ihrerseits hauptsächlich in 1- und 2-Stufen-Verfahren unterteilt werden.

Nach den Einstufen-Verfahren werden die freien Carbonsäuren mit geeigneten Fluorierungsmitteln direkt in die entsprechenden Carbonsäurefluoride überführt :

$$R{-}COOH \xrightarrow{\text{Fluorierungsmittel}} R{-}COF$$

(R = organischer Rest)

Die Eignung verschiedener Fluorierungsmittel für diesen Zweck wurde u. a. in der Arbeit von D. G. Cox et al., J. Fluorine Chemistry 23 (1983) 383-388, näher untersucht. Unter den geprüften Fluorierungsmitteln Schwefeltetrafluorid $SF_4$, Diethylamino-Schwefeltrifluorid $(C_2H_5)_2NSF_3$ Yarovenko-Reagenz $(C_2H_5)_2N{-}CF_2{-}CFClH$ Perfluoro-2-methyl-2-penten

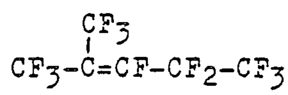

Fluorsulfonsäure $FSO_3H$ und dem Ishikawa-Reagenz $(C_2H_5)_2N{-}CF_2{-}CFH{-}CF_3$ wurde die letztgenannte Verbindung (Ishikawa-Reagenz) als am geeignetsten herausgestellt. Das Ishikawa-Reagenz muß jedoch erst hergestellt und destillativ gereinigt werden. Für die Herstellung (aus Diethylamin und Perfluorpropen) haben d. G. et al. a. a. O. folgende Reaktionsgleichung angegeben :

$$(C_2H_5)_2NH + CF_2 = CFCF_3 \longrightarrow$$

$$\longrightarrow (C_2H_5)_2NCF = CF{-}CF_3 + (C_2H_5)_2N{-}CF_2{-}CFH{-}CF_3$$

Die Ausbeute an dem Gemisch der beiden Reaktionsprodukte soll nach dem experimentellen Teil des vorerwähnten Artikels lediglich 65 % betragen.

Nach den gängigsten Zweistufen-Verfahren zur Herstellung von Carbonsäurefluoriden aus den zugrundeliegenden freien Carbonsäuren werden die Carbonsäuren in der ersten Reaktionsstufe zunächst in die Carbonsäurechloride überführt :

$$R{-}COOH \xrightarrow{\text{Chlorierungsmittel}} RCOCl$$

Als Chlorierungsmittel eigenen sich hier — etwa nach C. Ferri, Reaktionen der organischen Synthese, G. Thieme Verlag Stuttgart 1978, 460, sowie NS-A-1 921 767 nnd Houben-Weyl, Methoden der organischen Chemie, Bd. VIII/3, 472 (1952) - u. a. $SO_2 + Cl_2$, $SOCl_2$, $PCl_5$, $COCl_2$ und Benzotrichlorid $C_6H_5CCl_3$.

Beispielsweise aus Monochloressigsäure und Benzotrichlorid entsteht hiernach Chloracetylchlorid (und Benzoylchlorid) ohne Katalysator bei 100 bis 120 °C, in Gegenwart von $ZnCl_2$ bei etwas niedrigerer Temperatur (80 bis 90 °C) ; die zugrundeliegende Reaktionsgleichung ist :

$$ClCH_2{-}COOH + C_6H_5CCl_3 \xrightarrow{(ZnCl_2)} ClCH_2{-}COCl + C_6H_5COCl + HCl$$

In der zweiten Reaktionsstufe werden die Carbonsäurechloride dann mit Fluorierungsmitteln wie z. B. HF, KF, $KHF_2$, $KSO_2F$, NaF etc. — vgl. z. B. Houben-Weyl, Methoden der organischen Chemie, Bd. V/3 (1962), S. 119/20, 148-150 und 171-173 — umgesetzt :

$$RCOCl \xrightarrow{\text{Fluorierungsmittel}} RCOF$$

Für die Zweistufen-Verfahren ist vor allem die Notwendigkeit zweier Reaktionsstufen nachteilig.

Wenn man bei der vorerwähnten Umsetzung zwischen Carbonsäuren und Benzotrichlorid das Benzotrichlorid durch Benzotrifluorid ersetzt, erfolgt — wie eigene Versuche gezeigt haben — jedenfalls mit ω-H-Perfluorpropionsäure als Ausgangs-Carbonsäure nach mehrstündigem Erhitzen auf Temperaturen bis 190 °C in Abwesenheit von Katalysatoren keine Reaktion.

In Gegenwart von Katalysatoren ($TiO_2/TiCl_4$) reagiert — ebenfalls nach eigenen Versuchen — auch etwa Propionsäure $C_2H_5$—COOH mit Benzotrifluorid bei mehrstündigem Erhitzen auf 130 °C nicht.

Wegen des erhöhten Bedarfs an halogenierten aliphatischen Carbonsäurefluoriden sowie wegen der nicht bzw. nicht ganz befriedigenden einschlägigen Verfahren des Standes der Technik bestand die Aufgabe, ein verbessertes Verfahren zur Herstellung speziell solcher halogenierter aliphatischer Carbonsäurefluoride zu finden.

Diese Aufgabe konnte erfindungsgemäß durch die Umsetzung halogenierter aliphatischer Carbonsäuren mit Trihalogenmethylaromaten, welche in der Summe überwiegend oder ausschließlich F-Atome in den Trihalogenmethylgruppen enthalten, in Gegenwart von Lewis-Säuren als Katalysatoren gelöst werden. Erfindungsgegenstand ist daher ein Verfahren zur Herstellung halogenierter aliphatischer Carbonsäurefluoride durch Umsetzung halogenierter aliphatischer Carbonsäuren mit Fluorierungsreagenzien ; das Verfahren ist dadurch gekennzeichnet, daß man als Fluorierungsreagenzien Trihalogenmethylaromaten mit in der Summe überwiegend oder ausschließlich Fluoratomen in den Trihalogenmethylgruppen verwendet, wobei man die Trihalogenaromaten in einer zu den halogenierten aliphatischen Carbonsäuren mindestens etwa äquivalenten Menge einsetzt, und daß man die Umsetzung in Gegenwart von Lewis-Säuren als Katalysatoren durchführt.

Nach dem Verfahren werden in einer einfach durchzuführenden Einstufenreaktion aus halogenierten aliphatischen Carbonsäuren unter Einsatz technisch gut zugänglicher und preisgünstiger Fluorierungsreagenzien (Benzotrifluorid !) und Katalysatoren die entsprechenden halogenierten aliphatischen Carbonsäurefluoride in Ausbeuten von z. T. bis zu etwa 95 % d. Th. erhalten. Das Gelingen dieser Reaktion war außerordentlich überraschend, weil ω-H-Perfluorpropionsäure mit Benzotrifluorid bei mehrstündigem Erhitzen auf erhöhte Temperaturen in Abwesenheit von Katalysatoren nicht reagiert. Es war durchaus nicht zu erwarten, daß die Reaktion nur durch die Anwesenheit von Lewis-Säuren als Katalysatoren dann ohne weiteres zu dem entsprechenden Säurefluorid abläuft, zumal etwa die bekannte Reaktion zwischen Monochloressigsäure und Benzotrichlorid (vgl. Houben-Weyl, Bd. VIII/3, a. a. O.) sowohl in Abwesenheit als auch in Gegenwart eines Katalysators praktisch in gleicher Weise — lediglich bei geringfügig unterschiedlichen Temperaturen — abläuft.

Das Gelingen der erfindungsgemäßen Umsetzung war weiterhin auch deswegen noch überraschend, weil die — allerdings nicht halogenierte — Propionsäure mit Benzotrifluorid auch in Gegenwart von Lewis-Säure-Katalysatoren ($TiO_2/TiCl_4$ !) auch bei mehrstündigem Erhitzen auf 130 °C kein Propionylfluorid liefert.

Als halogenierte aliphatische Carbonsäuren können für das erfindungsgemäße Verfahren im Prinzip alle möglichen halogenierten unverzweigten oder verzweigten, gesättigten oder ungesättigten, ein- oder mehrbasischen aliphatischen Carbonsäuren eingesetzt werden. Bevorzugt ist jedoch der Einsatz halogenierter aliphatischer Carbonsäuren der Formel I :

$$X—R—COOH \qquad (I),$$

worin

R = 2-wertiger aliphatischer $C_1$-$C_{10}$-Rest, dessen H-Atome mindestens zur Hälfte durch Halogen — vorzugsweise F und/oder Cl — ersetzt sind,

und

X = H, Halogen — vorzugsweise F oder Cl, oder COOH.

Beispielhafte halogenierte aliphatische Carbonsäuren — und zwar sowohl bevorzugte als auch nicht bevorzugte — sind :

$CCl_3COOH$
$CF_2HCOOH$
$CHFClCOOH$
$CF_3$—COOH
$HCF_2CF_2COOH$
$H(CF_2)_4COOH$
$F_3CCF_2COOH$

$F_3C(CF_2)_5COOH$
$F_3C(CF_2)_6COOH$
$HOOC—(CF_2)_2—COOH$
$CF_2BrCOOH$
$F_3C—(CF_2)_{12}—COOH$
etc.

Als Trihalogenmethylaromaten mit — in der Summe — überwiegend oder ausschließlich Fluoratomen in den Trihalogenmethylgruppen werden bevorzugt Verbindungen der Formel II — allein oder in Mischung miteinander — verwendet :

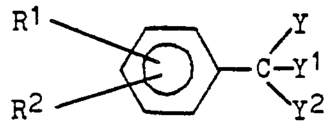

worin Y,
$Y^1$ und $Y^2$ = unabhängig voneinander F oder Cl,
$R^1$ und $R^2$ = ebenfalls unabhängig voneinander H, Halogen — vorzugsweise F oder Cl — oder

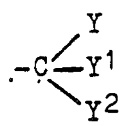 ,

wobei in der Summe aller vorhandenen

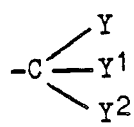 -

Gruppen mindestens doppelt soviel F- wie Cl-Atome vorliegen müssen.

Vorzugsweise ist in den Verbindungen der Formel II

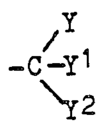 = $CF_3$ oder $-CF_2Cl$;

besonders bevorzugt ist

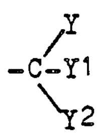 = $-CF_3$ und

$R^1$ und $R^2$ = H, F oder Cl.

Beispielhafte Verbindungen der Formel II sind :

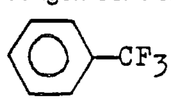

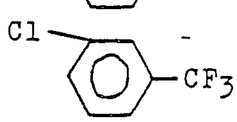

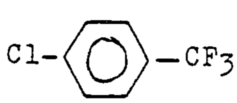

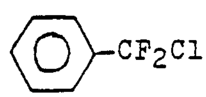

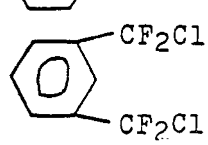

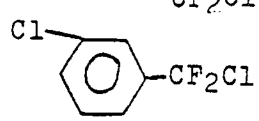

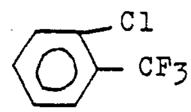

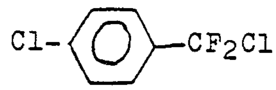

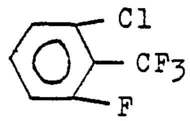

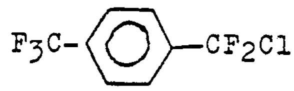

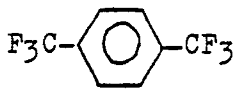

etc.

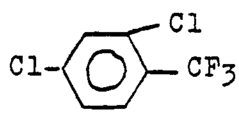

Besonders bevorzugte Einzelverbindung ist Benzotrifluorid.

Im Falle des Einsatzes von Mischungen von Trihalogenmethylaromaten kann z. B. auch ein fluorfreier Trihalogenmethylaromat wie z. B. Benzotrichlorid mitverwendet werden. Es ist nur immer darauf zu achten, daß dann auch soviel fluorhaltiger Trihalogenmethylaromat anwesend ist, daß in der Summe aller vorhandenen Triahlogengruppen überwiegend F-Atome vorliegen.

Benzotrifluorid sowie die anderen vorerwähnten Trihalogenmethylaromaten sind bekannte und z. T. auch im Handel erhältliche Produkte.

Die Fluorierungsreagenzien bzw. Trihalogenmethylaromaten mit in der Summe überweigend oder ausschließlich Fluoratomen in den Trihalogenmethylgruppen werden in einer zu den halogenierten aliphatischen Carbonsäuren mindestens etwa äquivalenten Menge, bevorzugt in einem etwa 5- bis etwa 50-%igen Überschuß, verwendet. Ein höherer Überschuß ist gegebenenfalls nur dann notwendig, wenn die eingesetzte halogenierte aliphatische Carbonsäure einen zu hohen Anteil an Wasser enthält. Eine « äquivalente Menge » bedeutet pro Carboxylgruppe der halogenierten aliphatischen Carbonsäure eine Trihalogenmethylgruppe in den Trihalogenmethylaromaten. Die zu beispielsweise einem Mol ω-H-Perfluorpropionsäure äquivalente Menge Benzotrifluorid ist ein Mol, entsprechend der Reaktionsgleichung :

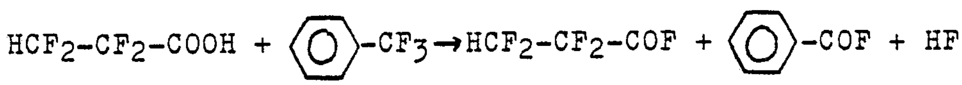

Geeignete Katalysatoren für das erfindungsgemäße Verfahren sind ganz allgemein Lewis-Säuren. Bevorzugt sind die Oxide und/oder Halogenide und/oder Alkoholate der folgenden Elemente : Cu, Zn, Hg, B, Al, Sn, Ti, Zr, As, Sb, V, Fe und Ni. Von den Halogeniden sind wiederum die Chloride und Fluoride bevorzugt ; bevorzugte Alkoholate sind die niederen Ester (insbes. Methyl- und Ethylester) der Borsäure. Die Katalysatoren können sowohl einzeln als auch in Mischung miteinander verwendet werden. Bevorzugt sind Mischungen der Oxide mit dem Chlorid oder Fluorid jeweils des gleichen Elements. Die Ester der Borsäure werden bevorzugt allein verwendet. Die Katalysatormenge liegt im allgemeinen zwischen etwa 0,05 und etwa 25 Mol.-%, vorzugsweise zwischen etwa 0,2 und etwa 5 Mol-%, bezogen auf die halogenierte aliphatische Ausgangs-Carbonsäure.

Die Reaktionstemperatur liegt normalerweise zwischen etwa 20 und etwa 250 °C, vorzugsweise zwischen etwa 40 und etwa 200 °C, insbesondere zwischen etwa 60 und etwa 160 °C.

Die Reaktion kann prinzipiell bei Normal-, Unter- oder Überdruck durchgeführt werden. Bevorzugt ist die Umsetzung bei Normal- oder Überdruck. Wenn bei Normaldruck gearbeitet wird, ist es vorteilhaft, die entstehenden Niedrigsieder laufend abzutrennen, um die gewünschte Reaktionstemperatur halten zu können.

Es ist von Vorteil, während der gesamten Reaktionsdauer den Ansatz gut zu rühren und dadurch für eine gleichmäßige Durchmischung zu sorgen. Die Reaktionszeit beträgt im allgemeinen zwischen etwa 2 und etwa 60 Stunden, insbesondere zwischen etwa 3 und etwa 30 Stunden.

Nach einer beispielhaften Arbeitsweise für die Durchführung des erfindungsgemäßen Verfahrens gibt man die halogenierte aliphatische Carbonsäure, den bzw. die Trihalogenmethylaromaten und den Katalysator zusammen und erhitzt

a) in der Normaldruckvariante in einem Gefäß aus geeignetem Material (z. B. Polytetrafluorethylen, Metall, in selteneren Fällen Glas, etc.), bis Gasentwicklung eintritt. Niedrigsiedende Produkte gehen dabei zusammen mit dem gleichzeitig entstandenen Halogenwasserstoff in eine gekühlte Vorlage ab,

während höhersiedende Produkte weitgehend im Reaktionsgefäß bleiben.

b) In der Überdruckvariante werden die Ausgangsstoffe vorteilhaft in einem Autoklaven unter Eigendruck so lange auf Reaktionstemperatur erhitzt, bis der Druck über mehrere Stunden konstant bleibt. Niedrigsiedende Produkte werden zusammen mit dem entstandenen Halogenwasserstoff aus dem Autoklaven in ein gekühltes Metallgefäß abgegast und aufgearbeitet. Höhersiedende Produkte werden nach Zusatz eines Bindemittels für HF direkt aus dem Reaktionsgemisch abdestilliert.

Wenn das gebildete halogenierte aliphatische Carbonsäurefluorid nicht destillativ von gleichzeitig gebildeter Flußsäure abgetrennt werden kann, wird das Gemisch bevorzugt nach einer der beiden folgenden Methoden getrennt :

a) Absorption von HF an NaF :

Das rohe Reaktionsgemisch wird mit überschüssigem NaF versetzt, gerührt und anschließend destilliert. Dabei wird HF als $NaHF_2$ gebunden. Dies kann in Gegenwart oder Abwesenheit von inerten Lösemitteln wie z. B. Ethern, Nitrilen, Sulfonen etc. durchgeführt werden.

b) Abtrennung von HF mit Trialkylaminen :

Das rohe Reaktionsgemisch wird mit der berechneten Menge an Trialkylamin (etwa 1/3 Mol Trialkylamin pro Mol HF), z. B. Triethylamin, versetzt und destilliert. Das entstandene Addukt Trialkylamin · ca. 3HF bleibt als Hochsieder zurück. Durch Destillation kann das Addukt als hochwertiges Fluorierungsmittel rein gewonnen werden.

Die nachfolgenden Beispiele sollen das erfindungsgemäße Verfahren nun näher erläutern. Nach den (Erfindungs-) Beispielen folgen noch 2 Vergleichsbeispiele, aus denen hervorgeht, daß ω-H-Perfluorpropionsäure mit Benzotrifluorid auch bei mehrstündigem Erhitzen auf Temperaturen bis zu etwa 190 °C in Abwesenheit von Katalysatoren nicht reagiert (Vergleichsbeispiel 1) sowie daß auch zwischen (nicht halogenierter) Propionsäure und Benzotrifluorid in Gegenwart von $TiO_2$ und $TiCl_4$ als Katalysator bei mehrstündigem Erhitzen auf 130 °C ebenfalls keine Reaktion eintritt (Vergleichsbeispiel 2).

Alle in den (Erfindungs- und Vergleichs-) Beispielen eingesetzten Verbindungen waren von technischer Reinheit. Der Wassergehalt der Carbonsäuren lag im allgemeinen unter einem Gewichtsprozent.

A) Erfindungsbeispiele

Es werden hier lediglich ein Beispiel für die Arbeitsweise unter Normaldruck (Beispiel 1a) und ein Beispiel für die Arbeitsweise unter Überdruck (Beispiel 5a) detailliert beschrieben. Sämtliche übrigen Beispiele wurden analog durchgeführt ; die dazu noch wesentlichen Einzelheiten sind aus der nachstehenden Tabelle ersichtlich.

Beispiel 1

a) In einem Glaskolben mit Magnetrührer, Thermometer, Rückflußkühler und Kältefalle wurden 78 g (0.53 Mol)

$H—CF_2—CF_2—COOH$, 118 g (0.6 Mol)

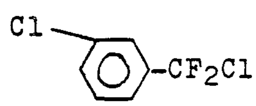

und 0.5 g (3.7 mMol) $ZnCl_2$ (wasserfrei) vorgelegt. Der Ansatz wurde erhitzt. Ab 60 °C setzte Gasentwicklung ein, die bei 120-130 °C kräftig wurde. Der Ansatz wurde erhitzt, bis die Sumpftemperatur 180 °C erreichte. In der Kältefalle (— 78 °C) befanden sich danach 65 g farblose Flüssigkeit, die einer Kältedestillation unterworfen wurde. Mit Kp 6-9 °C wurden 40 g (51 %) $H—CF_2—CF_2—COF$ erhalten.

Beispiel 5

a) Die Reaktion wurde in einem 5-Liter-Stahlautoklaven, der mit einem Hub-Schub-Rührer ausgestattet war, durchgeführt. Es wurde eine Mischung aus 1 500 g (10.27 Mol) $H—CF_2—CF_2—COOH$, 1 752 g (12 Mol)

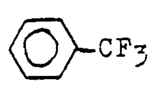,

5 g (62.5 mMol) $TiO_2$ und 5 g (26.3 mMol) $TiCl_4$ in den Autoklaven gegeben und unter Eigendruck auf

130 °C erhitzt. Bereits nach 7 Stunden Reaktionszeit war ein Druck von 21 bar erreicht, der nach weiteren 14 Stunden bei 130 °C nur noch auf 22,5 bar anstieg. Der Autoklav wurde auf 90 °C abgekühlt und über eine Kältefalle aus nichtrostenden Stahl entspannt, in der sich 546 g (13 Mol) NaF und 500 ml Diglyme befanden. Die Destillation des Falleninhaltes ergab 1 393 g (92 %) $H—CF_2—CF_2—COF$.

(Siehe Tabelle Seite 8 bis 13 f.)

Die Aufarbeitung des Säurefluorids erfolgte, wenn nicht anders angegeben, unter Zusatz von $(C_4H_9)_3N$.

1) Gasentwicklung ab 45 °C
2) Es wurden noch 10 % $H—CF_2—CF_2—COCl$ isoliert
3) Reindestillation ohne Zusatz von $(C_4H_9)_3N$
4) Reindestillation unter Zusatz von $NaF/CH_3CN$
5) Reindestillation unter Zusatz von NaF
6) Bereits nach 3 h erreicht
7) Reindestillation unter Zusatz von $(C_3H_7)_3N$
8) Bereits nach insgesamt 5 h erreicht
9) Nach 5 h : 17 bar
10) Nach 8 h erreicht
11) Nach 6 h : 40 bar
12) Das Reaktionsgemisch wurde mit NaF versetzt und destilliert
13) Destillation nach Einpumpen von

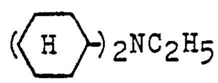

in den Autoklaven
14) Enthielt eine unbekannte Menge an Wasser

B) Vergleichsbeispiele

Vergleichsbeispiel 1

In einem 250 ml Autoklaven aus rostfreiem Stahl wurden 73 g (0.5 Mol) $H—CF_2—CF_2—COOH$ und 102 g (0.7 Mol)

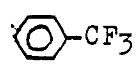

vorgelegt. Es wurde

16 Stunden auf 140 °C,
23 Stunden auf 170 °C und
5 Stunden auf 190 °C erhitzt.

Nach dem Abkühlen des Autoklaven auf Raumtemperatur war kein Druck mehr vorhanden. Im Reaktionsgemisch konnten kein HF und kein $H—CF_2—CF_2—COF$ nachgewiesen werden.

Vergleichsbeispiel 2

Im 1-Liter-Eisenautoklaven mit Hub-Schub-Rührer wurden 148 g (2 Mol) $CH_3—CH_2—COOH$, 321 g (2.2 Mol)

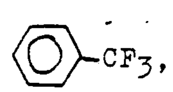

1 g (12.5 Mol) $TiO_2$ und 1 g (5.3 mMol) $TiCl_4$ 16 Stunden auf 130 °C erhitzt. Es stellte sich ein Druck von 2 bar ein. Die Aufarbeitung ergab keine Hinweis auf entstandenes $CH_3—CH_2—COF$.

Tabelle : Erfindungsbeispiele

| Bsp. Nr. | Reaktionsgefäß | X-R-COOH X-R-(Mol) | Trihalogenmethyl-aromat(en) (Mol) | | Katalysator (mMol) | | Reaktionstemperatur und -zeit | Druck | Ausbeute an X-R-COF % |
|---|---|---|---|---|---|---|---|---|---|
| 1a | Glaskolben | $H-CF_2-CF_2$ (0.53) | 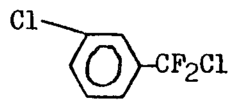 | (0.6) | $ZnCl_2$ | (3.7) | 120–130°C | drucklos | 51 % |
| 1b | Glaskolben | $H-CF_2-CF_2$ (0.5) | 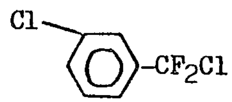 | (0.6) | $TiO_2$<br>$TiCl_4$ | (6.25)<br>(2.6) | 75°C 1) | drucklos | 57 % 2,3) |
| 2 | Polytetrafluorethylen-Kolben | $H-CF_2-CF_2$ (0.5) | 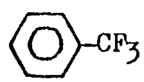 | (0.5) | $TiO_2$<br>$TiCl_4$ | (25)<br>(7.9) | | drucklos | 68 % 4) |
| 3 | Apparatur aus nichtrostendem Stahl | $H-CF_2-CF_2$ (2) | 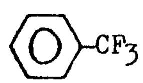 | (2) | $TiO_2$<br>$TiCl_4$ | (25)<br>(7.9) | 90 – 120°C | drucklos | 69 % 4) |
| 4 | wie in Beisp. 3 | $H-CF_2-CF_2$ (2) | 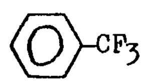 | (2.2) | $TiO_2$<br>$SbF_3$ | (25)<br>(11.2) | 100 – 125°C 4 h | drucklos | 44 % 5) |

Tabelle : Erfindungsbeispiele (Forts.)

| Bsp. Nr. | Reaktionsgefäß | X-R-COOH X-R-(Mol) | Trihalogenmethylaromat(en) (Mol) | Katalysator (mMol) | Reaktionstemperatur und -zeit | Druck | Ausbeute an X-R-COF % |
|---|---|---|---|---|---|---|---|
| 5a | Stahlautoklav mit Rührer | $H-CF_2-CF_2$ (10.27) | 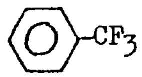 (12) | $TiO_2$ (62.5) $TiCl_4$ (26.3) | 130°C 21 h | 22,5 bar | 92 % |
| 5b | Stahlautoklav mit Rührer | $H-CF_2-CF_2$ (15) | 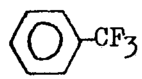 (17.55) | $TiO_2$ (62.5) $TiCl_4$ (26.3) | 130°C 16 h | 21 bar | 93 % |
| 5c | Stahlautoklav mit Rührer | $H-CF_2-CF_2$ (15) | 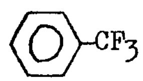 (17.55) 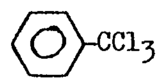 (0,1) | $TiO_2$ (62.5) $TiCl_4$ (26.5) | 130°C 19 h | 20 bar 6) | 94 % 7) |
| 5d | Stahlautoklav mit Rührer | $H-CF_2-CF_2$ (15) | 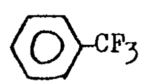 (17.55) 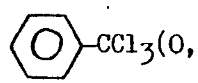(0,13) | $TiO_2$ (62.5) $TiCl_4$ (26.3) | 130°C 6 h | 20.5 bar | 90 % |
| 6 | Stahlautoklav mit Rührer | $H-CF_2-CF_2$ (2) | 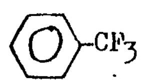 (2.2) | $TiO_2$ (12.5) | 130°C 19 h | 18 bar | 76 % 5) |

Tabelle : Erfindungsbeispiele (Forts.)

| Bsp. Nr. | Reaktions-gefäß | X-R-COOH X-R-(Mol) | Trihalogenmethyl-aromat(en) (Mol) | Katalysator (mMol) | Reaktions-temperatur und -zeit | Druck | Ausbeute an X-R-COF % |
|---|---|---|---|---|---|---|---|
| 7a | wie in Beisp. 5a | $H-CF_2-CF_2$ (2) | 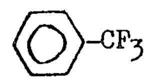 (2.2) | $Al_2O_3$ (49) | 130°C 66 h | 26 bar | 24 % 5) |
| 7b | wie in Beisp. 5a | $H-CF_2-CF_2$ (2) | 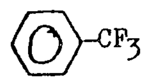 (2.2) 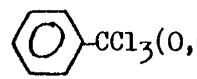(0,050) | $Al_2O_3$ (9.8) $AlCl_3$ (7.5) | 130°C 45 h | 17 bar | 63 % |
| 8 | wie in Beisp. 1 | $H-CF_2-CF_2$ (0.25) | 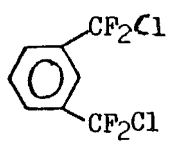 (0.15) | $TiO_2$ (12.5) $TiCl_4$ (5.3) | ~100°C | druck-los | 74 % 3) |
| 9 | wie in Beisp. 5a | $H-CF_2-CF_2$ (2) | 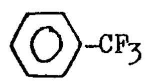 (1.47) 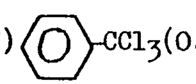(0.750) | $TiO_2$ (12.5) $TiCl_4$ (5.3) | 90°C, 2h und 130°C, 18h | (16bar) 35 bar8) | 75 % 4) |
| 10a | wie in Beisp. 5a | $H-CF_2-CF_2$ (2) | 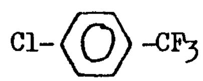 (2.2) | $TiO_2$ (12.5) $TiCl_4$ (5.3) | 130°C 18 h | 20 bar 9) | 77 % |
| 10b | wie in Beisp. 5a | $H-CF_2-CF_2$ (2) | 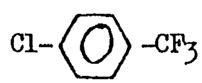 (2.2) | $TiCl_4$ (5.3) $TiF_4$ (8.1) | 130°C 20 h | 13 bar | 35 % |

Tabelle : Erfindungsbeispiele (Forts.)

| Bsp. Nr. | Reaktionsgefäß | X-R-COOH X-R-(Mol) | Trihalogenmethylaromat(en) (Mol) | Katalysator (mMol) | Reaktionstemperatur und -zeit | Druck | Ausbeute an X-R-COF % |
|---|---|---|---|---|---|---|---|
| 10c | wie in Beisp. 5a | $H-CF_2-CF_2$ (2) | 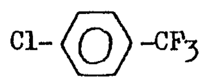 (2.2) | $TiO_2$ (12.5)<br>$TiF_4$ (8.1) | 130°C<br>20 h | 20 bar 10) | 77 % |
| 11 | wie in Beisp. 5a | $H-CF_2-CF_2$ (2) | 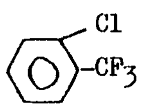 (2.2) | $TiO_2$ (12.5)<br>$TiCl_4$ (5.3) | 130°C<br>18 h | 20 bar | 77 % |
| 12 | wie in Beisp. 5a | $H-CF_2-CF_2$ (2) | 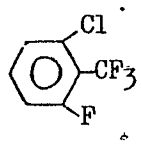 (2.2) | $TiO_2$ (12.5)<br>$TiCl_4$ (5.3) | 130°C<br>19 h | 14 bar | 43 % |
| 13 | wie in Beisp. 5a | $H-CF_2-CF_2$ (2) | 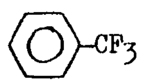 (2.2) 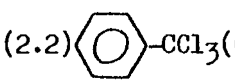 (0,050) | ZnO (12.3)<br>$ZnCl_2$ (7.4) | 130°C<br>45 h | 15 bar | 57 % |
| 14 | wie in Beisp. 5a | $H-CF_2-CF_2$ (2) | 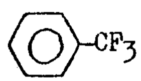 (2.2) 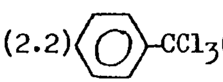 (0,050) | $SbCl_5$ (6.7) | 130°C<br>39 h | 18 bar | 67 % |
| 15 | wie in Beisp. 5a | $H-CF_2-CF_2$ (2) | 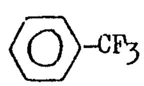 (2.2) 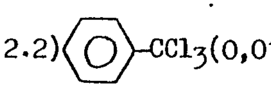 (0,010) | $TiO_2$ (12.5)<br>$SnCl_4$ (3.8) | 130°C<br>19 h | 16 bar | 70 % |

Tabelle : Erfindungsbeispiele (Forts.)

| Bsp. Nr. | Reaktionsgefäß | X-R-COOH X-R-(Mol) | Trihalogenmethylaromat(en) (Mol) | Katalysator (mMol) | Reaktionstemperatur und -zeit | Druck | Ausbeute an X-R-COF % |
|---|---|---|---|---|---|---|---|
| 16 | wie in Beisp. 5a | $H-CF_2-CF_2$ (1) | 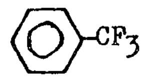 (1.1) 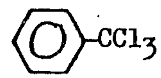 (0,026) | $Fe_2O_3$ (6.3)<br>$FeCl_3$ (6.2) | 130°C<br>15 h | 13 bar | 31 % |
| 17 | wie in Beisp. 5a | $H-CF_2-CF_2$ (2) | 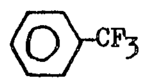 (2.2) 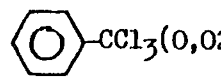 (0,026) | HgO (4.6)<br>$HgCl_2$ (3.7) | 130°C<br>18 h | 14 bar | 44 % |
| 18 | wie in Beisp. 5a | $H-CF_2-CF_2$ (1) | 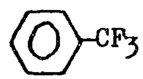 (1.1) 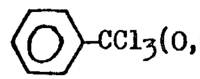 (0,013) | $TiO_2$ (12.5)<br>$NiCl_2$ (7.7) | 130°C<br>19 h | 18 bar | 64 % |
| 19 | wie in Beisp. 5a | $H-CF_2-CF_2$ (2) | 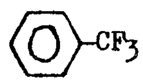 (2.2) 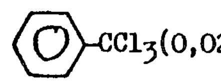 (0,026) | CuO (12.6)<br>$CuCl_2 \cdot 2H_2O$ (5.9) | 130°C<br>15 h | 14 bar | 43 % |
| 20 | wie in Beisp. 5a | $H-CF_2-CF_2$ (15) | 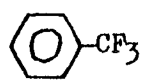 (17.55) | $B_2O_3$ (72) | 130°C<br>19 h | 24 bar | 89 % |
| 20a | wie in Beisp. 5a | $H-CF_2-CF_2$ (15) | 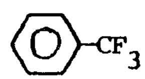 (18) | $B(OCH_3)_3$ (96) | 130°C<br>19 h | 20,5 bar | 84 % |
| 21 | wie in Beispiel 5a | $CF_3$ (5) | 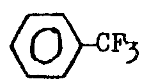 (5.4) | $TiO_2$ (25)<br>$TiCl_4$ (10.5) | 130°C<br>20 h | 44 bar 11) | 90 % 5) |

Tabelle : Erfindungsbeispiele (Forts.)

| Bsp. Nr. | Reaktions-gefäß | X-R-COOH X-R-(Mol) | Trihalogenmethyl-aromat(en) (Mol) | Katalysator (mMol) | Reaktions-temperatur und -zeit | Druck | Ausbeute an X-R-COF % |
|---|---|---|---|---|---|---|---|
| 22 | wie in Beisp. 5a | $CF_3-(CF_2)_6$ (0.7) | 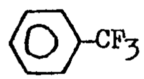 (0.7) | $TiO_2$ (12.5) $TiCl_4$ (5.3) | 130°C 20 h | 6 bar | 60 % 12) |
| 23 | Glaskolben | $CF_3-(CF_2)_6$ (0.35) | 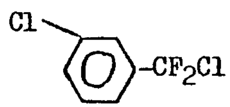 (0.45) | $TiO_2$ (6.25) $TiCl_4$ (2.6) | 120 -130°C | drucklos | 85 % 12) |
| 24 | wie in Beisp. 5a | CHFCl (2) | 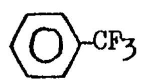 (2.2) | $TiO_2$ (12.5) $TiCl_4$ (5.3) | 130°C 18 h | 18 bar | 47 % 13) |
| 25 | Glaskolben | $CCl_3$ (0.4) | 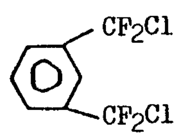 (0.22) | $TiO_2$ (12.5) $TiCl_4$ (5.3) | 50 - 90°C | drucklos | 70 % 12) |
| 26 | wie in Beisp. 5a | $HOOC-CF_2-CF_2$ 14) (~0.8) | 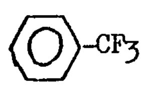 (2) 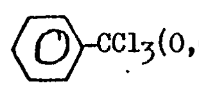 (0,050) | $TiO_2$ (12.5) $TiCl_4$ (5.3) | 130°C 20 h | 12 bar | 20 % |

## Patentansprüche

1. Verfahren zur Herstellung halogenierter aliphatischer Carbonsäurefluoride durch Umsetzung halogenierter aliphatischer Carbonsäuren mit Fluorierungsreagenzien, dadurch gekennzeichnet, daß man als Fluorierungsreagenzien Trihalogenmethylaromaten mit — in der Summe — überwiegend oder ausschließlich Fluoratomen in den Trihalogenmethylgruppen verwendet, wobei man die Trihalogenaromaten in einer zu den halogenierten aliphatischen Carbonsäuren mindestens etwa äquivalenten Menge einsetzt, und daß man die Umsetzung in Gegenwart von Lewis-Säuren als Katalysatoren durchführt.

2. Verfahren nach Anspruch 1, dadurch gekennzeichnet, daß man als halogenierte aliphatische Carbonsäuren Verbindungen der nachstehenden Formel I verwendet:

$$X—R—COOH \qquad (I)$$

worin

R = 2-wertiger aliphatischer $C_1$-$C_{10}$-Rest, dessen H-Atome mindestens zur Hälfte durch Halogen — vorzugsweise F und/oder Cl — ersetzt sind,

und

X = H, Halogen — vorzugsweise F oder Cl, oder COOH ist.

3. Verfahren nach den Ansprüchen 1 bis 2, dadurch gekennzeichnet, daß man als Fluorierungsreagenzien Trihalogenmethylaromaten der nachstehenden Formel II allein oder in Mischung miteinander verwendet:

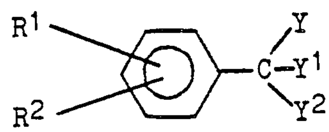

(II)

worin Y,

$Y^1$ und $Y^2$ = unabhängig voneinander F oder Cl,

$R^1$ und $R^2$ = ebenfalls unabhängig voneinander H, Halogen — vorzugsweise F oder Cl — oder

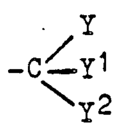

,

wobei in der Summe aller vorhandenen

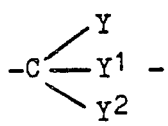

Gruppen mindestens doppelt soviel F- wie Cl-Atome vorliegen müssen.

4. Verfahren nach Anspruch 3, dadurch gekennzeichnet, daß in den Fluorierungsreagenzien der Formel II die

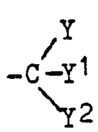

-Gruppe = $—CF_3$ oder $—CF_2Cl$ ist.

5. Verfahren nach Anspruch 3, dadurch gekennzeichnet, daß in den Fluorierungsreagenzien der Formel II die

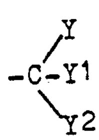

-Gruppe = $—CF_3$ und $R^1$ und $R^2$ = unabhängig voneinander H, F oder Cl sind.

6. Verfahren nach den Ansprüchen 1 bis 5, dadurch gekennzeichnet, daß man als Lewis-Säure-Katalysatoren die Oxide und/oder Halogenide und/oder Alkoholate der folgenden Elemente verwendet: Cu, Zn, Hg, B, Al, Sn, Ti, Zr, As, Sb, V, Fe und Ni.

7. Verfahren nach den Ansprüchen 1 bis 6, dadurch gekennzeichnet, daß man den Katalysator in einer Menge zwischen etwa 0,05 und etwa 25 Mol-%, vorzugsweise zwischen etwa 0,2 und etwa 5 Mol-%, bezogen auf die halogenierte aliphatische Ausgangs-Carbonsäure, einsetzt.

8. Verfahren nach den Ansprüchen 1 bis 7, dadurch gekennzeichnet, daß man die Umsetzung bei Temperaturen zwischen etwa 20 und etwa 250 °C, vorzugsweise zwischen etwa 40 und etwa 200 °C, insbesondere zwischen etwa 60 und etwa 160 °C, durchführt.

**Claims**

1. A process for the preparation of a halogenated aliphatic carboxylic acid fluoride by reacting a halogenated aliphatic carboxylic acid with a fluorinating reagent, which comprises using as fluorinating reagent a trihalogenomethyl aromatic compound containing, as a predominant total or exclusively, fluorine atoms in the trihalogenomethyl groups, the trihalogeno aromatic compound being used in an amount at least approximately equivalent to the halogenated aliphatic carboxylic acids, and carrying out the reaction in the presence of Lewis acids as catalysts.

2. The process as claimed in claim 1, wherein the halogenated aliphatic carboxylic acid used is a compound of the formula I below:

$$X\text{—}R\text{—}COOH \qquad (I)$$

in which R is a divalent aliphatic $C_1$-$C_{10}$ radical in which at least hafl of the H atoms have been replaced by halogen — preferably F and/or Cl — and X is H, halogen — preferably F or Cl, or COOH.

3. The process as claimed in claims 1 or 2, wherein the fluorinating reagents used are trihalogenomethyl aromatic compounds of the formula II below, on their own or as mixtures with one another:

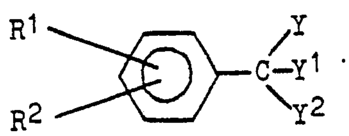

(II)

in which Y, $Y^1$ and $Y^2$, independently of one another, are F or Cl and $R^1$ and $R^2$, also independently of one another, are H, halogen — preferably F or Cl — or

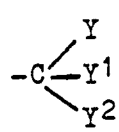

it being necessary for at least twice as many F atoms as Cl atoms to be present in the total of all the

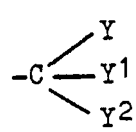

groups present.

4. The process as claimed in claim 3, wherein the

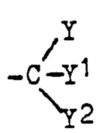 group

in the fluorinating reagent of the formula II is $—CF_3$ or $—CF_2Cl$.

5. The process as claimed in claim 3, wherein the

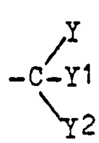 group

in the fluorinating reagent, of the formula II is $-CF_3$ and $R^1$ and $R^2$, independently of one another, are H, F or Cl.

6. The process as claimed in any of claims 1 to 5, wherein the Lewis acid catalyst used is an oxide and/or halide and/or alcoholate of the following elements : Cu, Zn, Hg, B, Al, Sn, Ti, Zr, As, Sb, V, Fe and Ni.

7. The process as claimed in any of claims 1 to 6, wherein the catalyst is employed in an amount between about 0.05 and about 25 mol%, preferably between about 0.2 and about 5 mol%, relative to the halogenated aliphatic carboxylic acid used as starting material.

8. The process as claimed in any of claims 1 to 7, wherein the reaction is carried out at temperatures between about 20 and about 250 °C, preferably between about 40 and about 200 °C, especially between about 60 and about 160 °C.

## Revendications

1. Procédé pour la préparation de fluorures d'acides carboxyliques aliphatiques halogénés, par réaction d'acides carboxyliques aliphatiques halogénés avec des réactifs de fluoration, caractérisé en ce qu'on utilise comme réactifs de fluoration des composés trihalogénométhylaromatiques comportant, au total, essentiellement ou exclusivement des atomes de fluor dans les groupes trihalogénométhyle, les composés trihalogénoaromatiques étant utilisés en une quantité au moins approximativement équivalente à celle des acides carboxyliques aliphatiques halogénés, et que l'on procède à la réaction en présence d'acides de Lewis servant de catalyseurs.

2. Procédé selon la revendication 1, caractérisé en ce qu'on utilise comme acides carboxyliques aliphatiques halogénés des composés ayant la formule I ci-après :

$$X-R-COOH \quad (I)$$

dans laquelle R est un radical aliphatique bivalent en $C_1$-$C_{10}$ dont les atomes d'hydrogène sont, au moins pour leur moitié, remplacés par un halogène — de préférence F et/ou Cl — et X = H, un halogène — de préférence F ou Cl, ou COOH.

3. Procédé selon les revendications 1 ou 2, caractérisé en ce qu'on utilise comme réactifs de fluoration des composés trihalogénométhylaromatiques ayant la formule II ci-après, seuls ou en mélange les uns avec les autres :

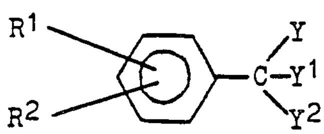

(II)

dans laquelle Y, $Y^1$ et $Y^2$, indépendamment les uns des autres, représentent chacun F ou Cl, $R^1$ et $R^2$, eux aussi indépendamment l'un de l'autre, représentent chacun H, un halogène, de préférence F ou Cl, ou

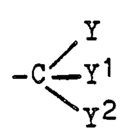

où, au total de tous les groupes

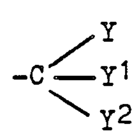

présents, le nombre d'atomes de F doit être au moins le double d'un nombre d'atomes de Cl.

4. Procédé selon la revendication 3, caractérisé en ce que, dans les réactifs de fluoration de formule II, le groupe

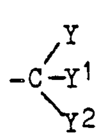

est $-CF_3$ ou $-CF_2Cl$.

5. Procédé selon la revendication 3, caractérisé en ce que, dans les réactifs de fluoration de formule II, le groupe

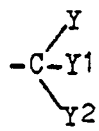

est —$CF_3$, et $R^1$ et $R^2$, indépendamment l'un de l'autre, sont chacun H, F ou Cl.

6. Procédé selon les revendications 1 à 5, caractérisé en ce qu'on utilise comme catalyseurs du type acide de Lewis les oxydes et/ou les halogénures et/ou les alcoolates des éléments suivants : Cu, Zn, Hg, B, Al, Sn, Ti, Zr, As, Sb, V, Fe et Ni.

7. Procédé selon les revendications 1 à 6, caractérisé en ce qu'on utilise le catalyseur en une quantité comprise entre environ 0,05 et environ 25 % en moles, de préférence entre environ 0,2 et environ 5 % en moles, par rapport à l'acide carboxylique aliphatique halogéné de départ.

8. Procédé selon les revendications 1 à 7, caractérisé en ce qu'on met en œuvre la réaction à des températures comprises entre environ 20 et environ 250 °C, de préférence entre environ 40 et environ 200 °C, en particulier entre environ 60 et environ 160 °C.